# EUROPEAN PATENT APPLICATION

(11) **EP 2 415 446 A2**
(43) Date of publication of application: **08.02.2012**
(21) Application number: 11173961.1
(22) Date of filing: 14.07.2011
(51) Int. Cl.: A61G 13/00, A61G 13/10, A61G 13/12, A61M 16/04

(54) **Cricoid manipulating apparatus for anesthesia assistance and tracheal intubation**

(30) Priority: 03.08.2010 US 370124 P; 08.10.2010 US 900653
(71) Applicant: Shimm, Peter, Chevy Chase MD 20815 (US)
(72) Inventor: Shimm, Peter, Chevy Chase MD 20815 (US)
(74) Representative: Johansson, Lars E.

(57) **Abstract**

A medical assistance apparatus, includes a support adjacent to a patient, a flexible shaft with a first end and a second end, with the first end being connected to the support, a housing connected to the second end of the flexible shaft, a shaft movably disposed with respect to the housing, an adjustment device associated with the housing, permitting adjustment of the shaft with respect to the housing, and a cricoid manipulating head connected to the shaft. The cricoid manipulating head is adjusted with respect to the housing along when the shaft is adjusted with respect to the housing.

## Description

### Cross-Reference to Related Application(s)

This application relies for priority on U.S. Provisional Patent Application Serial No. 61/370,124, filed on August 3, 2010, and U.S. Non-Provisional Patent Application Serial No. 12/900,653, filed on October 8, 2010, the contents of both of which are incorporated herein by reference.

### Field of the Invention

The present invention concerns an apparatus that may be employed in an operating room setting, among other environments. Specifically, the present invention concerns an apparatus that assists with the administration of anesthesia to a patient by providing cricoid manipulation. The apparatus assists with the application of anesthesia by helping an anesthesiologist to intubate a patient on an operating table, for example.

### Description of the Related Art

In an operating room, prior to an operation where anesthesia is administered to a patient, it is necessary to intubate the patient.

Intubation involves inserting an endotracheal tube ("ETT") in the patient's throat.

As should be apparent to those skilled in the art, including anesthesiologists, to insert the ETT, it is often necessary to manipulate the tissues in the patient's throat so that the ETT may be inserted without difficulty. For example, the practitioner will usually apply external pressure on the anterior portion of the patient's neck to push the tracheal opening in the throat (*i.e.,* the glottis) into a more posterior position. This places the throat into a better position for viewing though the mouth by the anesthesiologist using a laryngoscope.

Because the laryngoscope is held by the anesthesiologist's left hand, and placement of the ETT is performed by the right hand, it is often necessary to have a second person (*i.e.,* a nurse) assist the anesthesiologist to apply external pressure on the neck.

It is also desirable for an assistant (*i.e.*, a nurse) to be available for other tasks like holding the right side of the patient's mouth open, handing the anesthesiologist a suction tip or handing the anesthesiologist the ETT.

As may be apparent to those skilled in the art, when a nurse is asked to assist the anesthesiologist, the nurse is not available for other tasks in the operating room.

In addition, even after an anesthesiologist has manipulated the patient's throat to present an appropriate alignment for insertion of the ETT, it is seldom successful for the anesthesiologist to ask the nurse to take over by maintaining pressure on the patient's throat. Simply, a second person infrequently applies exactly the pressure that maintains the neck tissues in the optimal orientation. As may be appreciated by those skilled in the art, slight variations of pressure on a patient's throat can alter the alignment of the tissues to effectively prevent insertion of an ETT.

Cricoid manipulation is only one aspect of manipulation that may be employed by the anesthesiologist to assure proper throat alignment for proper administration of anesthesia during an operation.

For example, it is sometimes necessary to apply pressure to the person's forehead to maintain the head at a particular angular orientation. This may be required, for example, to assure adequate ventilation of the patient.

In addition, it may be necessary in some cases to apply an upward pressure on the back of the patient's neck to establish an air passageway that is suitable for a particular operation.

Further still, it may be necessary for the anesthesiologist to require that at least one of the cheeks of the patient be pulled laterally to facilitate access to the patient's air passageway.

In addition, it may be necessary to apply pressure to the patient's tongue to keep the tongue from interfering with the intubation procedure or the administration of anesthesia.

As should be apparent from the foregoing, positioning a patient properly before an operation often requires cooperation between two persons.

There are a number of devices that have been created to address various aspects associated with the administration of anesthesia and the intubation of patients. Some of those devices are discussed below.

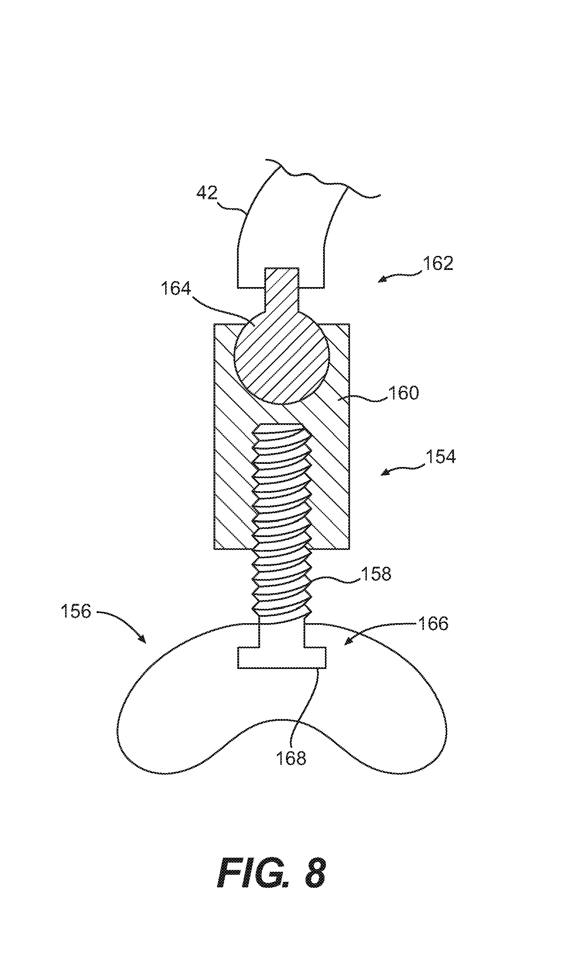

U.S. Patent Application Publication No. 2010/0089410 (hereinafter the '410 Application) describes an apparatus and a method for airway patency and head immobilization. The `410 Application describes a device that performs a jaw thrust and a chin tilt to open and maintain on opened passageway during emergency or surgical care. (The '410 Application at paragraph [0008].) With reference to Fig. 8, for example, the apparatus includes head support assemblies 140 and mandible rest assemblies 108. (The '410 Application at paragraph [0032].) The head rest assemblies 140 are adjustable until the head rests 150 contact the person's head. (The '410 Application at paragraph [0032].) A chin strap 122 is placed over the person's chin and cooperates with the mandible assemblies 108 to assist the health care provider with an advantageous jaw thrust maneuver. (The '410 Application at paragraph [0032].)

U.S. Patent Application Publication No. 2007/0181122 (hereinafter the '122 Application) describes an intubation positioning apparatus, breathing facilitator, and non-invasive assist ventilation device. The device is intended for use with patients who are lying down, are in a supine position, or are prone on an operating table. (The '122 Application at paragraph [0002].) The device includes a number of balloons 1-7 that may be inflated to elevate different parts of the human body, thereby altering the positional arrangement of various parts of the human body. (The '122 Application at paragraph [0015].)

U.S. Patent No. 7,621,009 (hereinafter the '009 Patent) describes a surgical coordinator for an anesthesiologist and methods of use. The device includes a flexible pad 21 with one or more holders 31 for holding fluid tubing lines leading to and from the patient during and after surgery. (The '009 Patent at col. 7, lines 17-21.)

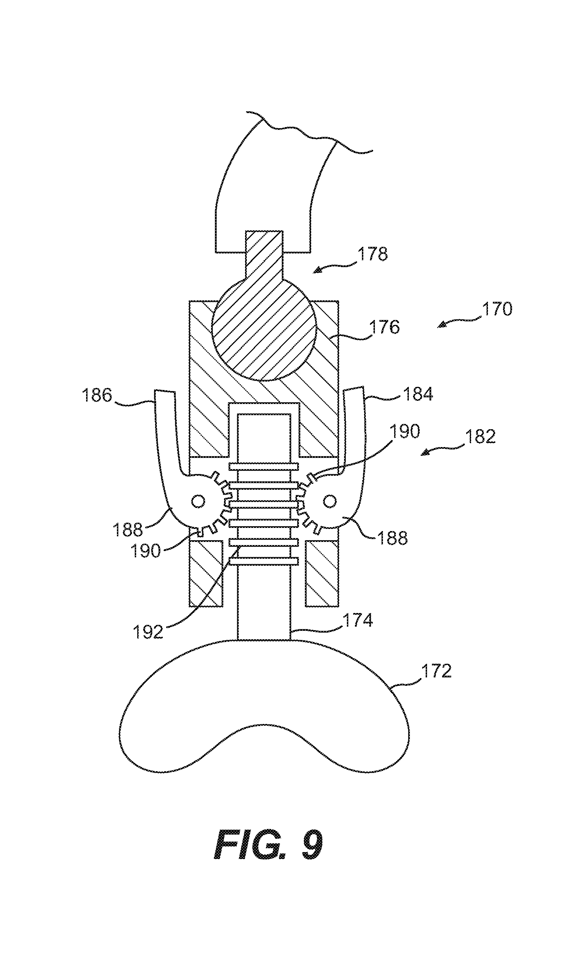

U.S. Patent No. 7,096,869 (hereinafter the '869 Patent) describes a device and method for maintaining a patient's airway. The device 10 described in the '869 Patent includes a neck support 11, an extendible swing arm 12, a cross-arm 13, and a chin support 14. (The '869 Patent at col. 2, lines 45-51.) As illustrated in Fig. 9, the device 10 lifts the patient's chin and tilts the patient's head. (The '869 Patent at col. 1, lines 46-49.)

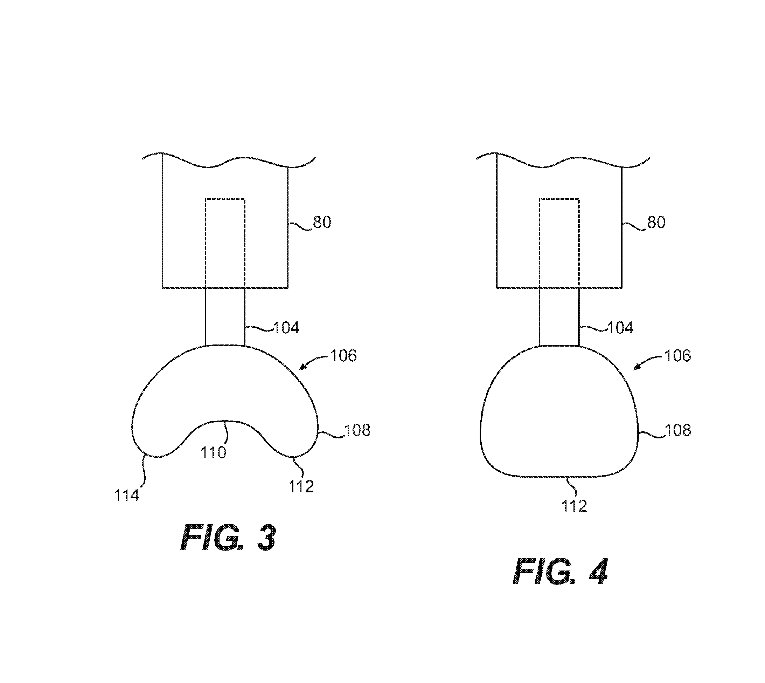

U.S. Patent No. 6,969,366 (hereinafter the '366 Patent) describes a hands-free chin lift and airway support device 10. The device 10 includes a chin rest 12, a base 16, and a shaft 14 connecting the chin rest 12 to the base 16. (The '366 Patent at col. 4, lines 39-41.) The shaft 14 is a cylindrical solid that is malleable to accommodate the person's anatomy. (The '366 Patent at col. 4, lines 56-58.) As illustrated in Figs. 3a and 3b, the base 16 is placed on the patient's manubrium 38 and is affixed to the patient via an adhesive 26. (The '366 Patent at col. 5, lines 2-7.) The device 10 holds the patient's head in the sniffing position. (The '366 Patent at col. 5, lines 22-27.)

U.S. Patent No. 6,935,340 (hereinafter the '340 Patent) describes a device that assists with endotracheal intubation. The endotracheal assistance device 100 is a pillow, cushion, support, or device that has at least two independently adjustable chambers 102, 104. (The '340 Patent at col. 3, lines 6-13.) The first chamber 102 supports the patient's shoulder region and the second chamber 104 supports the person's head. (The '340 Patent at col. 3, lines 14-20.) The first and second chambers 102, 104 cooperate with a pressure applicator 106 to position the person's head. (The '340 Patent at col. 3, lines 32-45.)

U.S. Patent No. 6,422,873 (hereinafter the '873 Patent) describes a device for use in the application of cricoid pressure and/or for training for such application. The background in the '873 Patent describes that studies have found that 47-61% of medical staff apply inadequate cricoid force during an intubation procedure before administration of anesthesia. (The '873 Patent at col. 1, lines 56-59.) The device includes a gauge 3 that indicates the amount of pressure (*i.e.,* force) applied to the cricoid cartilage. (The '873 Patent at col. 3, lines 57-66.)

U.S. Patent No. 6,171,314 (hereinafter the '314 Patent) describes a device to maintain open air passageways for people requiring anesthesia during a surgical procedure. (The '314 Patent at the Abstract.) The chin support 28 includes a central support 30 and to side supports 32, 34 that extend between the patient's chin and chest. (The '314 Patent at col. 4, lines 38-48.)

U.S. Patent No. 5,483,974 (hereinafter the '974 Patent) describes a device to apply, hold, and measure the cricoid pressure applied during endotracheal intubation. The '974 Patent describes how important it is to apply cricoid pressure to avoid aspiration of gastric contents during specific procedures. (The'974 Patent at col. 1, lines 45-48.) The device includes a housing 10 disposed on the cricoid cartilage 56. (The '974 Patent at col. 4, lines 40-44.) An inflatable bladder 40 applies pressure to the cricoid cartilage 56. (The '974 Patent at col. 4, lines 59-61.)

As should be apparent from the foregoing, there is considerable need for medical practitioners (*i.e.*, doctors, nurses, emergency medical technicians, etc.) to have available to them a wide variety of devices to assist with the administration of anesthesia and the intubation of patients.

These needs remain unaddressed by the prior art.

### Summary of the Invention

The present invention resolves one or more of the needs identified with respect to the related art and the prior art.

One embodiment of the present invention contemplates a medical assistance apparatus that includes a support adjacent to a patient, a flexible shaft with a first end and a second end, with the first end being connected to the support, a housing connected to the second end of the flexible shaft, a shaft movably disposed with respect to the housing, an adjustment device associated with the housing, permitting adjustment of the shaft with respect to the housing, and a cricoid manipulating head connected to the shaft. In this embodiment, the cricoid manipulating head is adjusted with respect to the housing along when the shaft is adjusted with respect to the housing.

It is also contemplated that the support for the medical device may be a rail connected to a patient table.

In one contemplated embodiment, the support is a rod that may be substantially vertically oriented adjacent to a table.

The flexible shaft may include at least one of a flexible material, a plurality of articulated segments, or a combination of a flexible material and at least one articulated segment.

It is contemplated that the medical device may have a ball joint connecting the flexible shaft to the housing, thereby permitting multi-axial movement of the housing with respect to the flexible shaft.

It is also contemplated that the adjustment device may have a plurality of tooth-engaging grooves disposed on the shaft, a gear rotationally disposed adjacent to and engaging the plurality of tooth-engaging grooves, a gear shaft on which the gear is disposed, the gear shaft being disposed rotationally with respect to the housing, and a head disposed on the gear shaft, permitting manipulation of the shaft and the gear, thereby causing movement of the shaft and the cricoid manipulating head with respect to the housing.

With respect to one embodiment of the present invention, the adjustment device may include threads on the shaft, a threaded opening in the housing into which the shaft is threadedly disposed, and a joint disposed at one end of the shaft, connecting the shaft to the cricoid manipulating head. In this embodiment, the joint permits the cricoid manipulating head to rotate with respect to the shaft, independently of any rotation of the shaft within the housing.

A further embodiment of the medical device contemplates that the adjustment device will have a plurality of grooves disposed on the shaft, a first adjustment lever and a second adjustment lever pivotally connected to the housing and disposed adjacent to substantially opposite sides of the shaft, rounded ends on each of the first and second adjustment levers, and a plurality of teeth disposed on the rounded ends of the first and second adjustment levers, the teeth engaging with the grooves on the shaft. Here, actuation of the first and second adjustment levers causes the shaft to move in relation to the housing.

The cricoid manipulating head may have a body with a hemi-cylindrical shape.

Alternatively, the cricoid manipulating head may have a body defined by a central region, a right side lobe, and a left side lobe, wherein the body compliments a natural shape of a person's neck.

In still another embodiment, the cricoid manipulator head may have a body defined by a central section with four flexible fingers extending therefrom.

The present invention also contemplates a medical suite with a medical assistance apparatus as discussed above and at least manipulator selected from a manipulator group. The manipulator group may include one or more of the following: (1) a cheek manipulator with a flexible shaft with a first end and a second end, the first end being connected to the support, an L-shaped body movably connected to second end of the flexible shaft, the L-shaped body having a first leg and a second leg, wherein the first leg is movably disposed with respect to the flexible shaft and the second leg is provided to engage a patient's cheek, and an adjustment device associated with the flexible shaft, permitting adjustment of the first leg of the L-shaped body with respect to the flexible shaft, (2) a tongue manipulator with a flexible shaft with a first end and a second end, with the first end being connected to the support, a clamp attached to the second end of the flexible shaft, the clamp having first and second clamping members that oppose one another, and a tongue depressor disposable between the first and second clamping members of the clamp, (3) a tool rack with a flexible shaft with a first end and a second end, with the first end being connected to the support, and a tool rack head disposed on the second end of the flexible shaft, wherein the tool rack head has a body with a plurality of flexible fingers attached thereto, the fingers being disposed adjacent to one another so that items may be inserted therebetween, the fingers including at least a flexible surface thereon so that items inserted therebetween are retained therebetween at least partially due to the flexible surface on the plurality of fingers, and (4) a neck manipulator having a flexible shaft with a first end and a second end, with the first end being connected to the support, and a neck manipulator head fixedly disposed on the second end of the flexible shaft.

One variation of the medical suite contemplates at least two or three manipulators are selected from the manipulator group defined above.

The present invention also contemplates a medical assistance device with a support adjacent to a patient, a flexible shaft with a first end and a second end, with the first end being connected to the support, an L-shaped body movably connected to second end of the flexible shaft, the L-shaped body having a first leg and a second leg, wherein the first leg is movably disposed with respect to the flexible shaft and the second leg is provided to engage a patient's cheek, and an adjustment device associated with the flexible shaft, permitting adjustment of the first leg of the L-shaped body with respect to the flexible shaft.

In still another contemplated embodiment, the medical device of the present invention includes a support adjacent to a patient, a flexible shaft with a first end and a second end, with the first end being connected to the support, a clamp attached to the second end of the flexible shaft, the clamp having first and second clamping members that oppose one another, and a tongue depressor disposable between the first and second clamping members of the clamp.

It is contemplated that the medical device of the present invention may include a support adjacent to a patient, a flexible shaft with a first end and a second end, with the first end being connected to the support, and a tool rack head disposed on the second end of the flexible shaft, wherein the tool rack head has a body with a plurality of flexible fingers attached thereto, the fingers being disposed adjacent to one another so that items may be inserted therebetween, the fingers having at least a flexible surface thereon so that items inserted therebetween are retained therebetween at least partially due to the flexible surface on the plurality of fingers.

The medical assistance device according to the present invention also may include a support adjacent to a patient, a flexible shaft with a first end and a second end, with the first end being connected to the support, and a neck manipulator head fixedly disposed on the second end of the flexible shaft, wherein pressure may be applied by the neck manipulator head to a posterior surface of a patient's neck.

In any of its embodiments, the medical assistance device also may be provided with a distal locking mechanism that permits a releasable locking of the flexible shaft to maintain the flexible shaft in a selected, predetermined orientation. The distal locking mechanism is contemplated to permit the practitioner to place the flexible shaft into a rigid condition with a one-handed operation, thereby facilitating manipulation of the flexible shaft.

Still further aspects of the present invention will become apparent from the discussion that follows and from the drawings appended hereto.

### Brief Description of the Drawings

The present invention will now be described in connection with several drawings, appended hereto, in which:

Fig. 1 is an elevational, end view of an operating table, illustrating various aspects of the present invention;

Fig. 2 is a side view illustration of a patient on an operating table, with the cricoid manipulating device of the present invention being illustrated in relation to the patient's neck;

Fig. 3 is a front view of a first embodiment contemplated for the cricoid manipulator of the present invention;

Fig. 4 is a side view of the cricoid manipulating device illustrated in Fig. 3;

Fig. 5 is a front view of a second embodiment of a cricoid manipulator contemplated for use with the present invention;

Fig. 6 is a bottom view of the cricoid manipulator illustrated in Fig. 5;

Fig. 7 is a side view illustration of the cricoid manipulator of the present invention, showing a first embodiment of an adjusting device associated therewith;

Fig. 8 is a side view illustration of the cricoid manipulator of the present invention, showing a second embodiment of an adjusting device associated therewith;

Fig. 9 is a cross-sectional, side view illustration of the cricoid manipulator of the present invention, showing a third embodiment of an adjusting device associated therewith;

Fig. 10 is a top view illustration of a cheek manipulator according to the present invention, shown in association with the mouth of a patient;

Fig.11 is a side view illustration of the cheek manipulator according to the present invention, which is illustrated in Fig. 10;

Fig. 12 is a front view of a tongue manipulator according to the present invention;

Fig. 13 is a top view of the tongue manipulator illustrated in Fig. 12;

Fig. 14 is a side view illustration of a patient's head, showing an application of pressure to the patient's forehead and chin via the cricoid manipulator of the present invention;

Fig. 15 is an elevational, end view of an operating table, illustrating various aspects of the present invention, including the use of the cricoid manipulator as a support for anesthesia gases;

Fig. 16 is a side view of a tool rack according to the present invention;

Fig. 17 is an end view of the tool rack depicted in Fig. 16;

Fig 18 is a side view of one contemplated embodiment of the flexible shaft portion of the present invention; and

Fig. 19 is a side view of another contemplated embodiment of the flexible shaft of the portion of the present invention.

### Detailed Description of Embodiment(s) of the Invention

The present invention will now be described in connection with one or more specific, contemplated embodiments. While specific embodiments are discussed, the present invention is intended to encompass equivalents and variations, as would be understood by those skilled in the art. In other words, the invention is not intended to be limited solely to the select embodiments described and/or depicted.

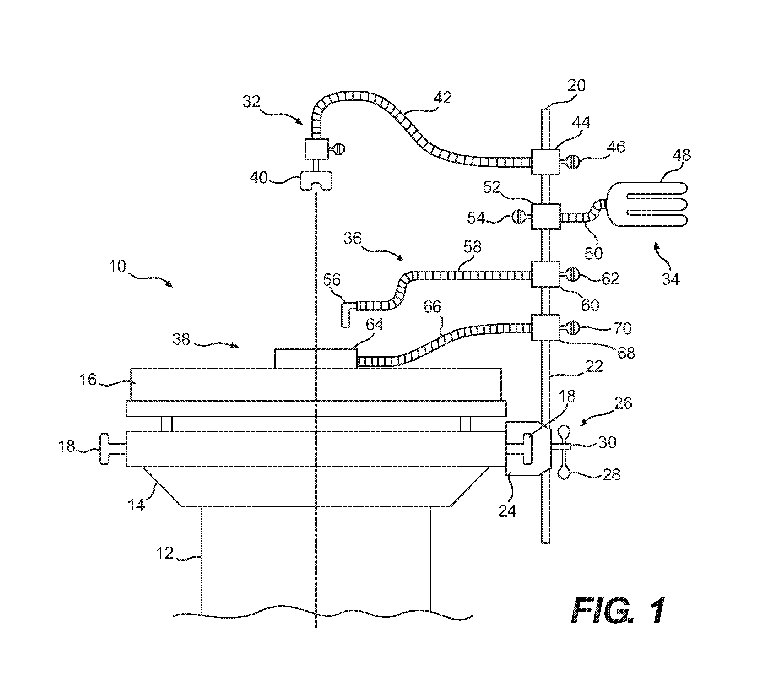

Fig. 1 is an end view representation of a patient table, such as an operating table 10 that incorporates many of the features of the present invention. As illustrated, the operating table 10 includes a pedestal 12 that supports an operating surface 14. A patient support 16 is disposed on the operating surface 14.

On either side of the operating surface 14, equipment rails 18 are provided. The equipment rails 18 typically extend the entire length of the operating surface 14. Of course, as should be apparent to those skilled in the art, other configurations also are known and are contemplated to fall within the scope of the present invention.

In the illustrated embodiment of the operating table 10, the equipment rails 18 are illustrated as T-shaped rails that extend laterally adjacent to the operating surface 14. As should be apparent, other types of equipment rails 18 (*i.e.,* other than T-shaped rails) may be used without departing from the scope of the present invention. It is noted that equipment rails 18, regardless of their specific cross-section or design, typically permit devices to be attached thereto along the length of the operating table 10. This permits equipment to be connected to the operating table 10 at any discrete location along its length.

In the embodiment illustrated in Fig. 1, a single equipment rack 20 is attached to the equipment rail 18 on the right-hand side of the drawing. The equipment rack 20 in this embodiment includes a rod 22 that connects to a clamp 24. The clamp 24 is intended to slide onto the equipment rail 18 from one end. So that the equipment rack 20 cannot be moved from the selected location on the equipment rail 18, the clamp 14 is provided with a fastener 26.

In the illustrated embodiment, the rod 22 may be a cylindrical metal rod. As should be apparent to those skilled in the art, the rod 22 need not be cylindrical. Moreover, the rod 22 need not be made from metal. Other configurations and materials may be employed without departing from the scope of the present disclosure.

In Fig. 1, the fastener 26 is a crew-type fastener. In other words, as the user twists a handle 28, a threaded rod 30 associated therewith is tightened against the equipment rail 18, which secures the clamp 24 in the location selected by the user.

As should be apparent, the simple construction for the clamp 24 is not intended to be limiting of the instant disclosure. The clamp 24 may be of any type and be made from any material suitable for the operating theater.

As shown in Fig. 1, there are a number of devices that are connected to the rod 22. The top-most device is referred to as the cricoid manipulator 32. The second device is a tool rack 34. The third device is a cheek manipulator 36. The fourth device is a neck manipulator 38.

The cricoid manipulator 32 includes a cricoid manipulator head 40, a flexible shaft 42, and a clamp 44 with an adjustment screw 46. The tool rack 34 includes a tool rack head 48, a flexible shaft 50, and a clamp 52 with and adjustment screw 54. Similarly, the cheek manipulator 36 includes a cheek manipulator head 56, a flexible shaft 58, and a clamp 60 with an adjustment screw 62. Finally, the neck manipulator 38 includes a neck manipulator head 64, a flexible shaft 66, and a clamp 68 and adjustment screw 70.

The details of the cricoid manipulator 32, the tool rack 34, the cheek manipulator 36, and the neck manipulator 38 are discussed in the paragraphs that follow.

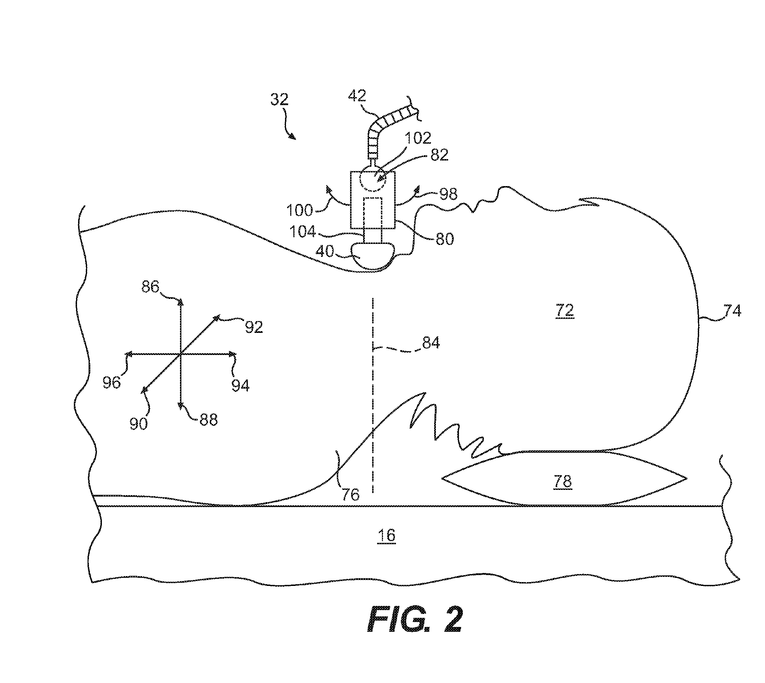

Fig. 2 illustrates a first embodiment of the cricoid manipulator 32 in relation to a patient 72 lying on the patient support 16 of the operating table 10. In the illustration, the patient's head 74 and neck 76 are shown. The patient's head 74 is positioned on a pillow 78 for illustrative purposes.

As should be appreciated by those skilled in the art, the cricoid cartilage is a ring of cartilage that surrounds the trachea. During intubation with an ETT, an anesthesiologist is required to apply pressure to the cricoid cartilage so that the tissues in the patient's throat are properly aligned for insertion of the ETT. The precise pressure applied to the cricoid cartilage differs for each patient.

It is noted that, while the present invention is contemplated for use by an anesthesiologist in an operating room, the present invention is not limited solely to this use. In addition, while aspects of the present invention are discussed in connection with intubation of a patient with an ETT, the present invention is not intended to be limited solely to this use.

As should be apparent, there are many medical practitioners who may benefit from aspects of the present invention in many different environments, too numerous to list here. The precise use or environment where the present invention is employed, therefore, is not considered to be critical to the present invention or to be limiting of the present invention in any manner.

In the paragraphs that follow, the term practitioner is employed to refer generally to any person that may rely on the present invention for any purpose. A practitioner may include, but is not limited to, a doctor, nurse, doctor's assistant, anesthesiologist, emergency medical technician, etc.

Returning to Fig. 2, once the appropriate pressure is applied to the cricoid cartilage, the anesthesiologist usually inserts the ETT with his or her free hand while maintaining the throat tissue in the appropriate orientation. This presents a challenge to the practitioner who may need both hands to guide the ETT into position. As such, many practitioners enlist assistance from another person to maintain pressure on the cricoid cartilage. This also presents a challenge since the assistant cannot visualize the throat tissue and, therefore, cannot typically apply appropriate pressure to the patient's neck in exactly the manner desired by the practitioner.

The cricoid manipulator 32 provides the "extra hand" that the practitioner requires for insertion of the ETT.

As illustrated in Fig. 2, in its simplest deployment, the cricoid manipulator 32 includes a housing 80 that is connected to the flexible shaft 42 via a ball joint 82. The ball joint 82 permits the housing 80 to be disposed in any number of angular orientations with respect to a vertical axis 84, which also is illustrated in Fig. 2.

With respect to the flexible shaft 42, it is contemplated that the flexible shaft will be made from one or more materials. It is contemplated that the shaft will be made from a material that allows the practitioner to bend the shaft 42 into any shape or configuration that may be useful in treating a patient 72. The flexible shaft 42 may be a continuous shaft construction. Alternatively, the shaft 42 may be constructed from a plurality of discrete segments that are connected to one another. In still another construction, the flexible shaft 42 may combine both continuous segments and discrete segments that move with respect to one another. The exact configuration for the flexible shaft 42 is not critical to the construction or operation of the present invention.

While not required for operation of the cricoid manipulator 32, multi-axial movement of the housing 80 is preferred because it allows the practitioner to apply pressure to the cricoid cartilage both in an anterior/posterior direction, illustrated by the arrows 86, 88, in a lateral direction (arrows 90, 92), and also in head to toe directions (arrows 94, 96). While the primary force that is applied is in the posterior direction 88, it is often necessary to apply pressure slightly to the patient's left (arrow 90) or right (arrow 92) to along the throat tissue as required for insertion of the ETT. It may also be necessary to apply pressure in a direction toward the patient's head (arrow 94) or toward the patient's toes (arrow 96). Any other combination of vectors also may need to be employed.

As noted above, the ball joint 82 permits multi-axial movement of the housing 80 in arcuate directions, such as those indicated by arrows 98, 100 in Fig. 2. In the illustrated embodiment, the ball joint 82 includes a spherical body 102 embedded in the housing 80 so that the housing 80 rotates about the spherical body 102. The spherical body 102, in turn, is fixedly connected to the flexible shaft 42.

In the embodiment illustrated in Fig. 2, the spherical body 102 may be made from stainless steel. As should be appreciated by those skilled in the art, however, the spherical body 102 may be made from any suitable material including aluminum, metal, plastic, or composites, among others. The housing 80 is anticipated to be constructed from a plastic material, but also may be made from any suitable material such as steel, stainless steel, aluminum, or composites, among others.

It is anticipated that the spherical body 102 will reside in a socket in the housing 80 in a tightly-fitted manner so that the angular orientation of the housing is maintained even after the practitioner releases the housing 80. Alternatively, the ball joint 82 may be loosely fitted but provided with a fastener or clamp (or other type of fixing device) that holds the housing 80 in a fixed orientation after being released by the practitioner. Since the exact construction for the ball joint 82 is not critical to operation of the cricoid manipulator 32, further details concerning this aspect of its construction are not provided.

The cricoid manipulator head 40 is disposed at one end of a shaft 104 that is movably inserted into the housing 80. In the illustrated embodiment, the shaft 104 is disposed within the housing so that the shaft 104 maintains its axial orientation with respect to the housing 80. In other words, the shaft 104 and the housing 80 are coaxially arranged with respect to one another. This construction is considered to be sufficient for operation of the cricoid manipulator 32 primarily because the ball joint 32 provides multi-axial movement of the cricoid manipulator head 40.

It is noted that the cricoid manipulator head 40 need not be connected fixedly to the shaft 104. To the contrary, any other type of connection may be employed. For example, the cricoid manipulator head 40 may be connected to a ball joint at the end of the shaft 104. Alternatively still, the cricoid manipulator head 40 may be pivotally disposed on the shaft 104. The exact connection between the cricoid manipulator head 40 and the shaft 104 is not critical to the operation of the cricoid manipulator 32.

The shaft 104 is axially moveable within the housing 80. This permits the practitioner to make minor adjustments of the cricoid manipulator head 40 with respect to the housing 80. Since the shaft 104 is moveable with respect to the housing 80, it is anticipated that the shaft will include a fixing element that will hold the shaft 104 in a particular position, once selected. Various contemplated embodiments of such a fixing device are discussed below.

It is contemplated that the cricoid manipulator head 40 may be constructed in any of a number of different shapes and sizes. As should be apparent, the exact dimensions and shapes of the cricoid manipulator head 40 are not critical to operation of the cricoid manipulator 32. It is noted that the cricoid manipulator head 40 is illustrated as a hemi-cylinder (or one half of a cylinder split along its axis).

Fig. 3 is a front view depicting one contemplated shape for the cricoid manipulator head 106 of the present invention. In this embodiment, the cricoid manipulator head 106 includes a body 108 with a central section 110 and right and left side lobes 112, 114. The shape of the cricoid manipulator head 108 has been selected to compliment the natural shape of the patient's neck 76 at the location of the cricoid cartilage. Other shapes may be employed without departing from the scope of the present invention.

It is contemplated that the central section 110 and the right and left lobes 112, 114 of the cricoid manipulator head 106 will be made from a flexible material such as rubber or foam so that the cricoid manipulator head 106 molds, at least partially, to the exterior surface of the patient's neck 76. Other materials, as should be apparent to those skilled in the art, also may be employed without departing from the scope of the present invention.

Fig. 4 is a side view of the cricoid manipulator head 106 illustrated in Fig. 3. As should be apparent from Figs. 3 and 4, the housing 80 and the shaft 104 are the same as depicted and described in connection with Fig. 2.

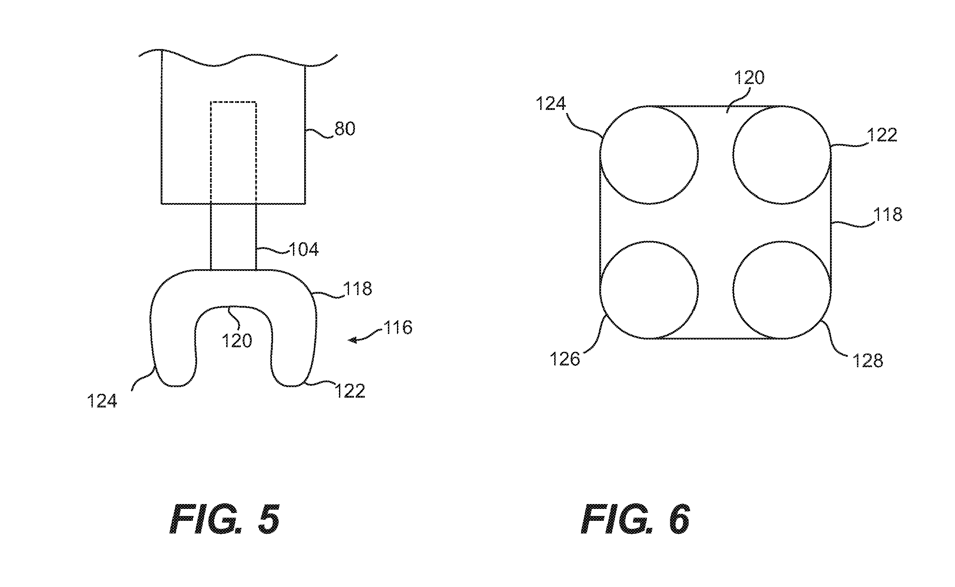

Fig. 5 is a front view of a second embodiment of a cricoid manipulator head 116 according to the present invention. The cricoid manipulator head 116 includes a body 118 with a central section 120 and four fingers 122, 124, 126, 128 extending therefrom. Fig. 6 provides a bottom view of the cricoid manipulator head 116, showing in greater detail the four fingers 122, 124, 126, 128.

As in the prior embodiment, the cricoid manipulator head 116 is intended to be made from a suitable, flexible material such as rubber or foam, among other possible materials. The fingers 122, 124, 126, 128 are anticipated to flex outwardly to accommodate the shape of the patient's neck 76.

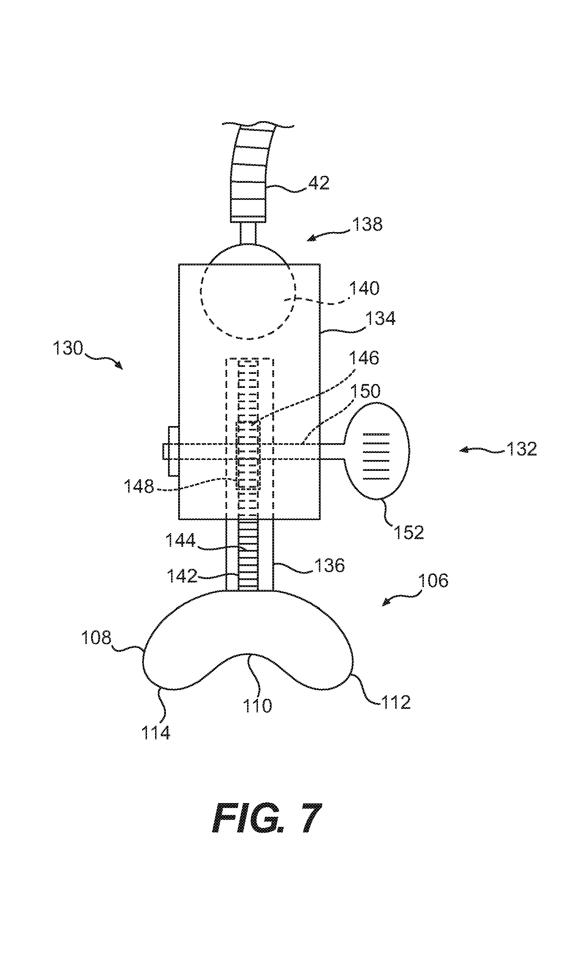

Fig. 7 is a side view of an embodiment of a cricoid manipulator 130 with an adjustment mechanism 132 (also referred to as a fixing device, herein), which is disposed within the housing 134. The cricoid manipulator 130 includes the cricoid manipulator head 106 described in connection with Figs. 3 and 4. The cricoid manipulator head 106 is connected to a shaft 136 that slides axially within the housing 134. So that the housing 134 is adjustable in multiple axial directions, the housing 134 is connected by a ball joint 138 to the flexible shaft 42. Like the ball joint 82, the ball joint 138 includes a spherical ball 140 disposed within a socket within the housing 134.

So that the practitioner may adjust the position of the cricoid manipulator head 106, the shaft 136 is provided with a central track 142 containing a plurality of teeth-engaging grooves 144. The grooves 144 extend from one end of the track 142 to the other. The grooves 144 mesh with teeth 146 on a gear 148 disposed on an adjustment shaft 150 within the housing 134. As illustrated, the adjustment shaft 150 extends through the housing 134. One end of the adjustment shaft 150 is provided with a key head 152 that may be manipulated by a practitioner. As should be apparent to those skilled in the art, by turning the key head 152, the practitioner turns the gear 148. The teeth 146 of the gear 148, in turn, engage the plurality of grooves 144. As a result, when the gear 148 is turned, the shaft 136 is moved relative to the housing 134.

In this embodiment, it is contemplated that the shaft 136 will be disposed in the housing 134 in such a manner that upward pressure on the shaft 136 will not cause the shaft 136 to be pushed into the housing 134 after release of the key head 152. Alternatively, it is contemplated that the adjustment mechanism may be provided with a locking device to hold the shaft 136 in a particular position relative to the housing 134, once the appropriate position is selected.

Fig. 8 is a side view, cross-sectional illustration of another type of adjustment mechanism 154 contemplated for use with a cricoid manipulator head 156.

In Fig. 8, the adjustment mechanism includes a threaded shaft 158 that engages a threaded opening in the housing 160. The threaded shaft 158 may be manipulated by the practitioner to move shaft 158 in relation to the housing 160. Specifically, the practitioner need but to rotate the shaft 158 with respect to the housing 160. In this regard, it is anticipated that the individual threads on the threaded shaft 158 will be spaced apart from one another a sufficient distance to permit relatively quick adjustment upon rotation of the shaft 158. In other words, a finely threaded shaft 158, while useable, would lend itself to small variations in the displacement of the shaft 158, which may not be the most practical. Closely-positioned threads would necessitate considerable rotation by the practitioner, which might frustrate the practitioner.

As in prior embodiments, the housing 160 connects to the flexible shaft 42 via a ball joint 162. The ball joint 162 includes a spherical body 164 that is embedded in a socket in the housing 160. The ball joint 162 permits multi-axial movement of the housing 160 with respect to the flexible shaft 42.

So that the cricoid manipulating head 156 is able to maintain a stable orientation with respect to the patient's neck 76, the shaft 158 is provided with a rotational joint 166 at one end. The rotational joint 166 includes a T-shaped structure 168 that is embedded within the cricoid manipulating head 156. The T-shaped structure 168 is a flat, circular body that permits the cricoid manipulating head 156 to rotate therearound. As a result, when the shaft 158 is rotated, the cricoid manipulating head 156 may be kept in its initially-selected position so that it does not rotate together with the threaded shaft 158.

Fig. 9 illustrates yet another contemplated embodiment for the cricoid manipulator 170 of the present invention. The cricoid manipulator 170 includes a cricoid manipulator head 172 connected to a shaft 174. In this embodiment, the cricoid manipulator head 172 is fixedly attached to the shaft 174. However, as in the embodiment illustrated in Fig. 8, the cricoid manipulator head 172 may be rotationally mounted onto the shaft 174.

The cricoid manipulator 170 includes a housing 176 that connects to the flexible shaft 42 via a ball joint 178. As in prior embodiments, the ball joint 178 includes a spherical body 180 that is embedded or inserted into the housing 176. The spherical body 180 permits the housing 176 to be positioned multi-axially.

An adjustment mechanism 182 in the housing 176 includes a first adjustment lever 184 and a second adjustment lever 186. The first adjustment lever 184 is positioned on one side of the housing 176 and the second adjustment lever 186 is positioned on the opposite side. The first and second adjustment levers 184, 186 include rounded ends 188 with teeth 190 that engage grooves 192 on the shaft 174. By pressing on the levers 184, 186, the practitioner causes the teeth 190 to engage the grooves, thereby forcing the shaft 174 out from the end of the housing 176. The levers 184, 186 may be spring actuated so that release of the levers 184, 186 causes the levers 184, 186 to remain engaged with the grooves 192 on the shaft 174, thereby holding the shaft 174 in a fixed position with respect to the housing 176. By completely depressing the levers 184, 186 against the housing 176, the teeth 190 may be disengaged from the grooves 192 so that the shaft 174 retracts into the housing 176. The shaft 174 may be spring-biased into a retracted state within the housing 176.

As should be appreciated by those skilled in the art, there may be additional structures that may be employed in connection with the adjustment mechanism 182 to permit the practitioner to extend and/or retract the shaft 174 in the housing 176. The exact details of the adjustment mechanism 182 is not critical to the present invention. As a result, further details are not provided herein.

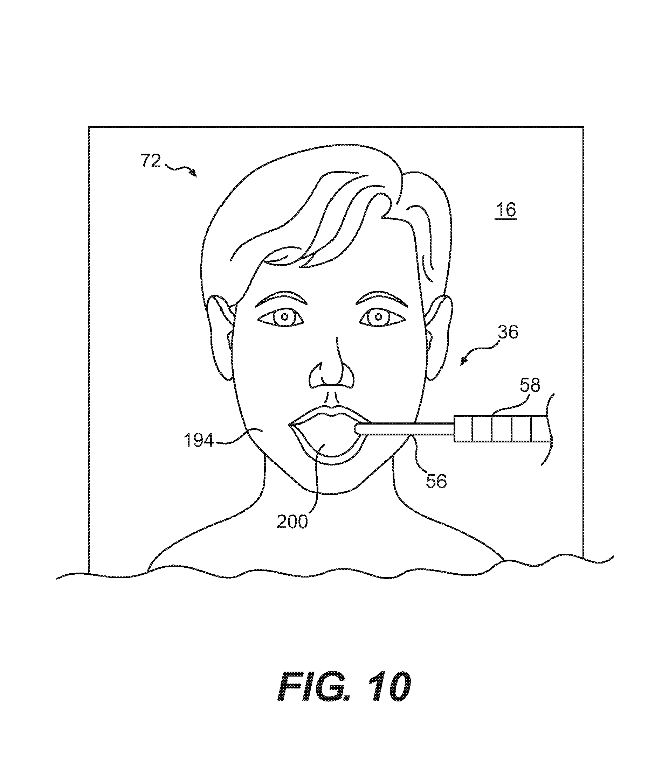

Fig. 10 is a top view of a patient 72 on the patient support 16 of an operating table 10. This illustration provides a top view of another aspect of the present invention, the cheek manipulator 36. The cheek manipulator 36 is provided so that the practitioner may have assistance in holding at least one of the cheeks 194 of the patient 72 in an extended position to facilitate insertion of an ETT into the patient's throat.

The cheek manipulator 36 includes the cheek manipulating head 56 attached to the flexible shaft 58. The flexible shaft 58 is intended to be of the same construction as the flexible shaft 42.

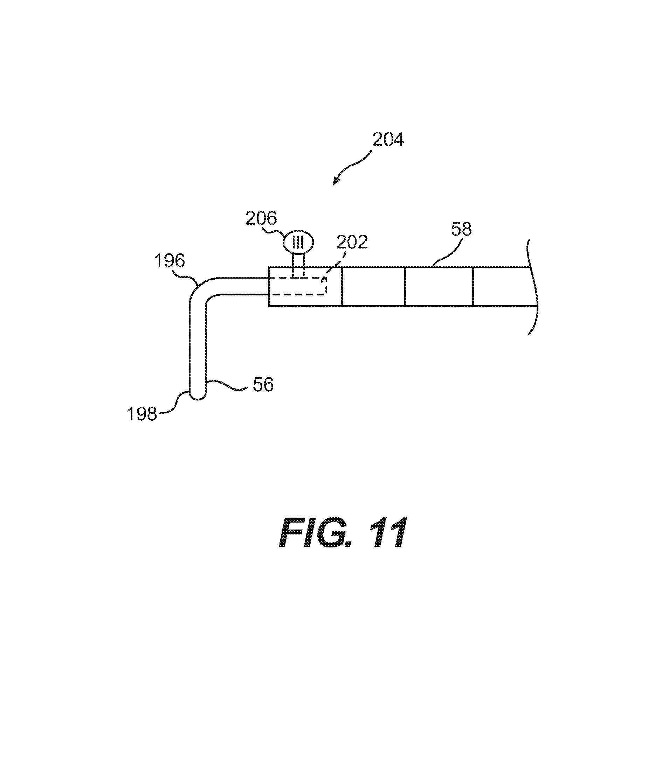

Fig. 11 illustrates the cheek manipulator 36 of the present invention in greater detail. The cheek manipulating head 56 includes an L-shaped body 196 that is connected to the flexible shaft 58. The L-shaped body 194 includes a first end 198 that is inserted into the patient's mouth 200. A second end 202 of the L-shaped body 196 is positioned within the flexible shaft 58.

So that the L-shaped body 196 may be adjusted in association with the flexible shaft 58, an adjustment mechanism 204 is provided. The adjustment mechanism 204 includes a threaded key 206. The threaded key 206 may be rotated by the practitioner to loosen and/or tighten its grip against the second end 202 of the L-shaped body 196. This permits the L-shaped body 196 to be adjusted within the flexible shaft 58.

It is contemplated that the L-shaped body 196 will be cylindrical in cross-section. As a result, not only may the second end 202 be adjusted in its lateral position with respect to the flexible shaft 58, but it also may be rotated within the flexible shaft 58. This provides the practitioner with greater adjustability. As should be apparent to those skilled in the art, a cylindrical cross-section is not required to practice the present invention. It is also noted that the L-shaped body 196 may be made from a material such as stainless steel. Alternatively, the L-shaped body 196 may be made from plastic of other suitable, disposable (or recyclable) material. The exact material is not critical to the present invention.

In one alternative embodiment, it is contemplated that the L-shaped body 196 may be held in a housing that is connected to the flexible shaft 58. If a housing (not shown) is employed, the housing may be adjustable positionally with respect to the flexible shaft 58. Other embodiments also are contemplated for the cheek manipulator 36.

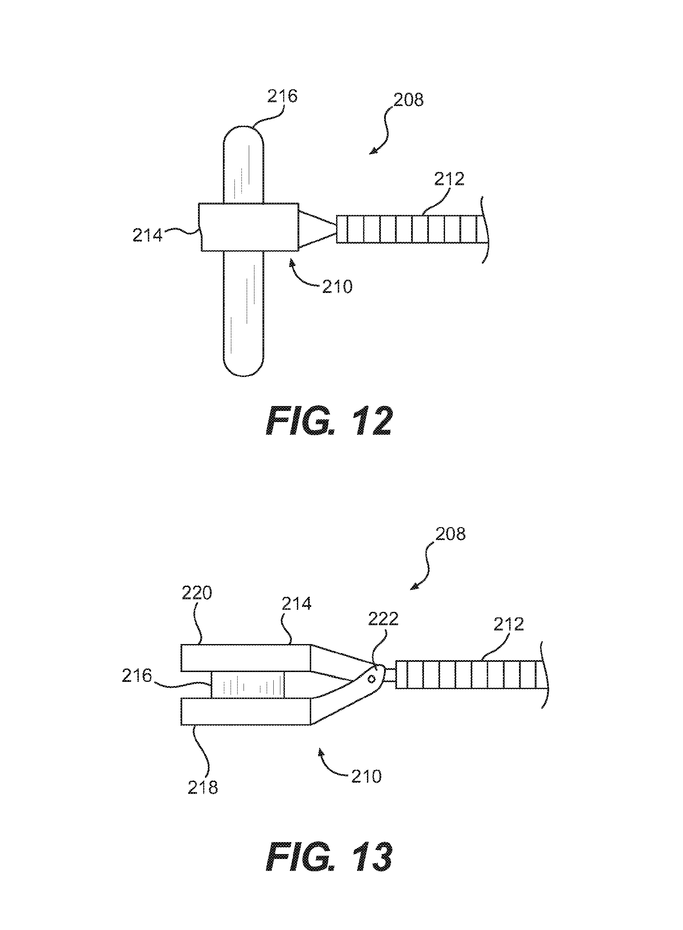

Fig. 12 is a front view of a tongue manipulator 208 according to the present invention. The tongue manipulator 208 includes a tongue manipulating head 210 that is connected to a flexible shaft 212. The tongue manipulating head 210 includes a clamp 214 that holds a tongue depressor 216 therein.

Fig. 13 is a top view of the tongue manipulator 208 of the present invention. Details of the clamp 214 are more evident in this illustration than in Fig. 12. The clamp 214 includes a first clamping member 218 and a second clamping member 220. The first and second clamping members 218, 220 are connected to one another via a joint 222 such that the members 218, 220 oppose one another in a scissor action. The first and second clamping members 218, 220 are contemplated to be biased, via a spring (not shown), so that they apply clamping pressure to the tongue depressor 216.

As should be apparent to those skilled in the art, when utilized, the clamp 214 holds the tongue depressor 216 securely so that the tongue depressor may be inserted into the patient's mouth 200 and hold the patient's tongue in a position that assists the practitioner to insert an ETT into the patient's throat.

With respect to the tongue depressor 216, it is contemplated that a traditional tongue depressor 216 will be used. A traditional tongue depressor 216 is a popsicle-stick-shaped, flat, wooden device that doctors traditionally use in an examining room. As should be apparent, other types and shapes of tongue depressors 216 may be employed with the tongue manipulator 208 without departing from the scope of the present invention.

As also may be appreciated by those skilled in the art, the tongue manipulator 208 may have any other type of construction that permits application of pressure to the patient's tongue.

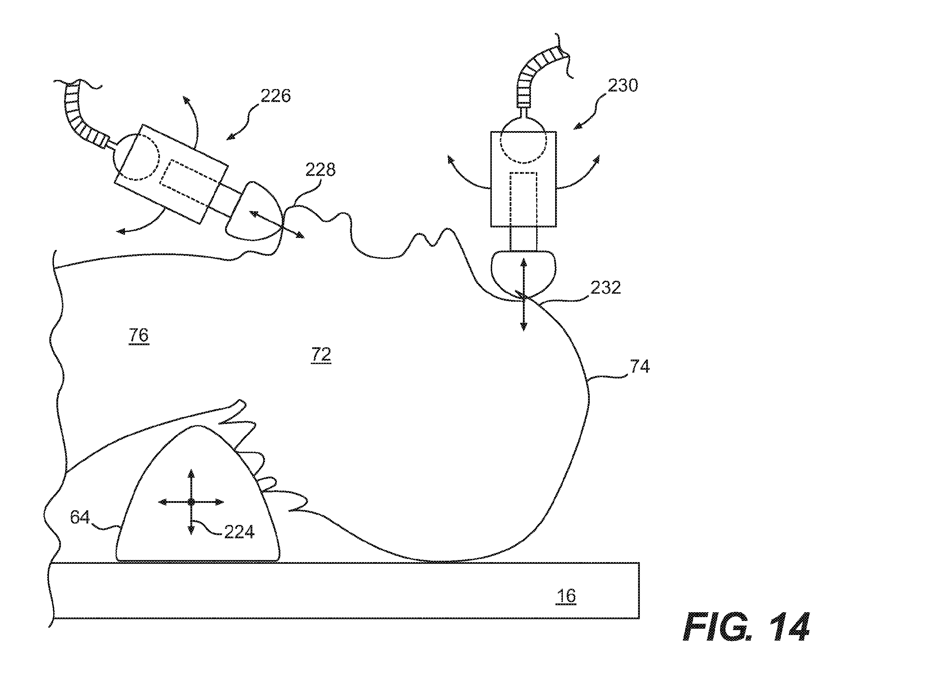

Fig. 14 illustrates further aspects of the present invention, the neck manipulator 38. The neck manipulator includes a neck manipulator head 64 that is connected to a flexible shaft 66. The neck manipulator head 64 may be adjusted in any number of different orientations, as illustrated by the arrows 224. The neck manipulator head 64 is provided so that the practitioner may apply pressure to the posterior portion of the patient's neck 76. This may be desirable in instances where the practitioner needs to tilt the patient's head 74, for example.

Fig. 14 also illustrates other aspects of the present invention. Specifically, multiple cricoid manipulators 32 may be used to assist with positioning the patient's head 74 for intubation.

In Fig. 14, a first cricoid manipulator 226 (which has the construction of the cricoid manipulator 32 described above) is positioned to support the patient's chin 228. A second cricoid manipulator 230 is provided to apply pressure to the patient's forehead 232. Together with the neck manipulator 38, the two cricoid manipulators 226, 230 help to hold the patient's head 74 so that the practitioner may intubate the patient 72. As may be apparent, each of the multiple cricoid manipulators 32 may be connected to the equipment rack 20 that is illustrated in Fig. 1. Alternatively, the cricoid manipulators 32 may be affixed directly to the equipment rail 18.

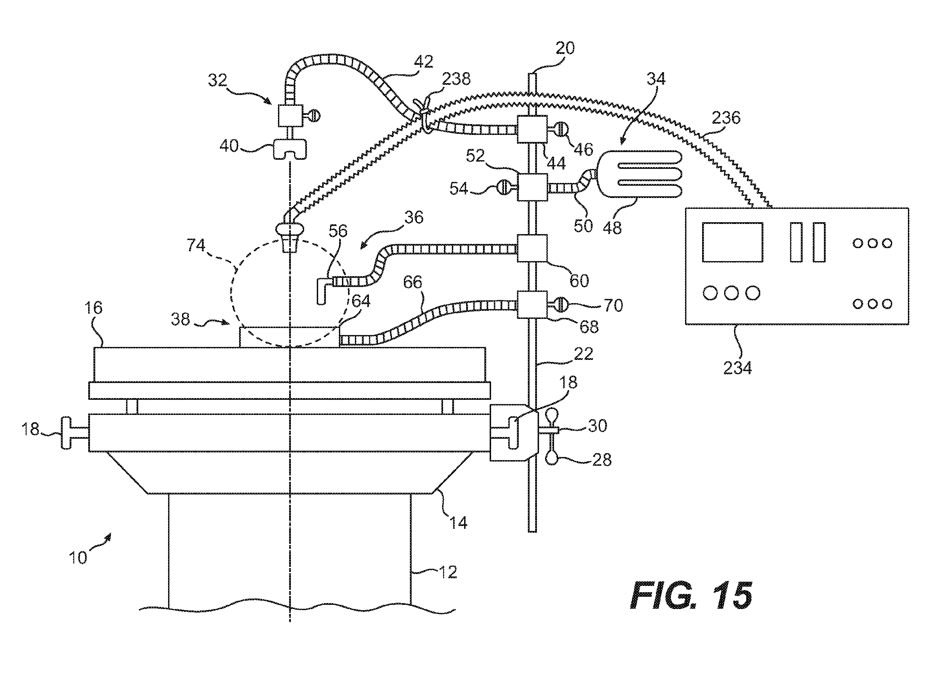

Fig. 15 illustrates yet another aspect of the present invention. In this figure, an anesthesia administering device 234 is illustrated adjacent to the operating table 10. The device 234 is connected to a tube 236 that provides the anesthetic gas to the patient. One or more of the flexible shafts, such as the flexible shaft 42, may be used to support the tube 236. In the example illustrated in Fig. 15, a clamp 238 secures the tube 236 to the flexible shaft 42. In one contemplated embodiment, the clamp 238 may be a strap made from a hook and loop fastening material. One trademark for this type of material is Velcro®. Alternatively, the clamp 238 may be a traditional operating room clamp. Other types of clamps may be employed without departing from the scope of the present invention.

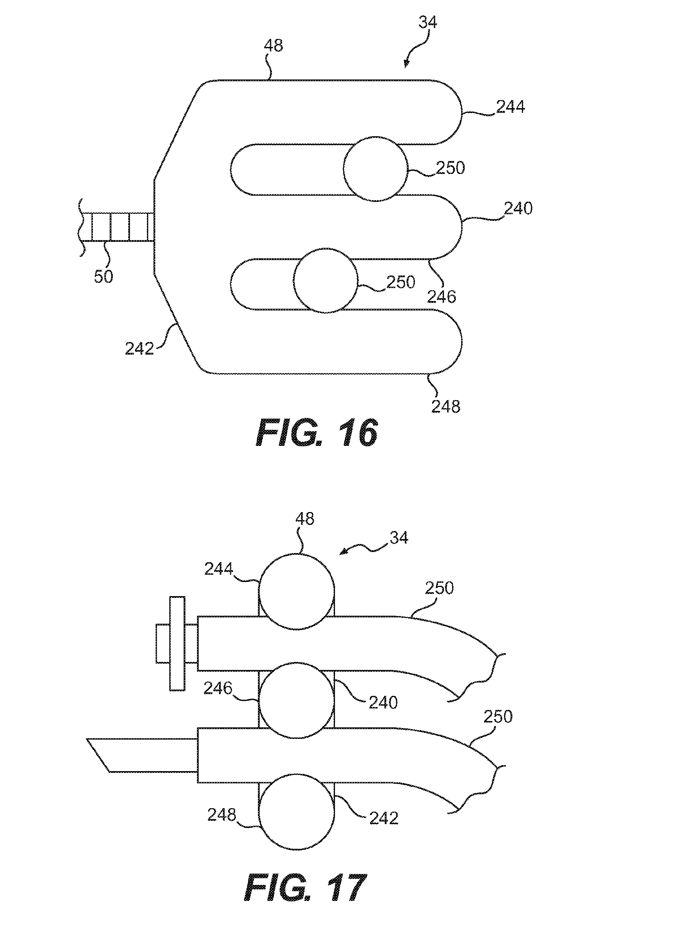

Fig. 16 is a front view of still another aspect of the present invention. Fig. 16 illustrates the tool rack 34 with the tool rack head 48 attached to the flexible shaft 50.

The tool head 48 includes an E-shaped body 240. As such, there is a root segment 242 from which three fingers 244, 246, 248 extend. Tools 250 may be placed between and be gripped by the fingers 244, 246, 258, as illustrated. The tools 250 may be tubes, surgical devices, or any other type of equipment that the practitioner may wish to have handy. One contemplated tool 250 could be a suction tip, for example.

So that the tools 250 are secured in the fingers 244, 246, 248, it is contemplated that the fingers 244, 246, 248 are constructed with a flexible surface, such as a foam surface. In one contemplated construction, the fingers 244, 246, 248 may include rigid interior members that for a "skeleton" within the tool head 48. The foam may be molded over the skeleton. As such, when the practitioner inserts a tool 250 between the fingers 244, 246, 248, the foam deforms and holds the tool in place.

As should be appreciated by those skilled in the art, the tool head 48 may have any type of construction that would permit grasping of one or more tools 250. In addition, while three fingers 244, 246, 248 are illustrated, the fool head 48 may include a greater or fewer number of fingers 244, 246, 248. In addition, while the fingers 244, 246, 248 are illustrated as linear structures, they may take any suitable shape.

With renewed reference to Fig. 1, it is noted that the rod 22 may have any length suitable for the operating room. It is contemplated, however, that the rod 22 will extend about 2 feet (be about 60 cm) above the surface of the patient support 16. In addition, any number of manipulators may be connected to the rod 22.

It is noted that any of the adjusting mechanisms described herein may be utilized with any of the manipulators also described herein.

A discussion of various aspects of the present invention is now provided in the context of use from the viewpoint of an anesthesiologist in an operating room.

Prior to use, the flexible shaft 42 keeps the cricoid manipulator 32 out of the way of the patient's head 74. With the cricoid manipulator 32 out of the way, the practitioner has room to insert a laryngoscope into the patient's throat. The laryngoscope (not illustrated) is inserted into the patient's mouth 200 with the anesthesiologist's left hand.

While viewing into the mouth 200, the anesthesiologist bends the flexible shaft 42 down to the neck 76 with the right hand. The anesthesiologist applies downward force on the neck 76 with the cricoid manipulator head 40 in order to achieve an optimal view of the glottis. This may require manipulation of the articulated joint(s) (*i.e.,* the ball joint 82) on the cricoid manipulator 32 to shift the glottis to either side. Once a good view of the glottis is achieved, the flexible shaft 42 is released. It is possible that the flexible shaft 42 may retract a bit when it is released and the view of the glottis may worsen. If so, the cricoid manipulator head 40 may be adjusted via the adjustment mechanism 132, for example. The extension of the shaft 136 to press the cricoid manipulator head 108 on the flexible shaft 42 compensates for any retraction of the flexible shaft 42 when it is released.

With respect to the cheek manipulator 36, it is anticipated that the L-shaped body 196 may be a fully bendable structure to accommodate a particular patient's physiology.

The cheek manipulator 36 may be used in the following manner. As before, the laryngoscope is inserted into the patient's mouth with the anesthesiologist's left hand. While viewing into the mouth, the anesthesiologist inserts the cheek manipulator 36 into the patient's mouth 200. The cheek manipulator 36 holds the patient's cheek 194 to one side to afford a more clear view of the patient's throat. The tongue manipulator 208 may be used in conjunction with the cheek manipulator 36, as needed. One inserted into the patient's mouth, it is anticipated that the cheek manipulator 36 will remain in place, because the elastic force of the cheek 194 on the cheek manipulator 36 will be minimal.

The tool rack 34 may be used by the anesthesiologist to hold various devices such as the endotracheal tube ("ETT"), a suction tip, a topical lidocaine injector, etc. The tool head 48 is anticipated to have soft fingers 244, 246, 248. The tools 250 would be inserted, like a cigarette, between the fingers 244, 246, 248.

The tool rack 34 might be used as follows. Before laryngoscopy, the flexible shaft 50 is bent to a convenient position so the anesthesiologist may easily grasp the tools 250 grasped by the tool head 48. The various items like the ETT, the suction tip, topical local anesthetic injector, Magill Forceps, etc., are wedged between the fingers 244, 246, 248. During larynoscopy, the anesthesiologist may grasp any of the tools 250 without diverting attention from the task at hand. Tools 250 may then be returned to the fingers 244, 246, 248 so that the tools 250 are accessible at a later point in the procedure and/or operation.

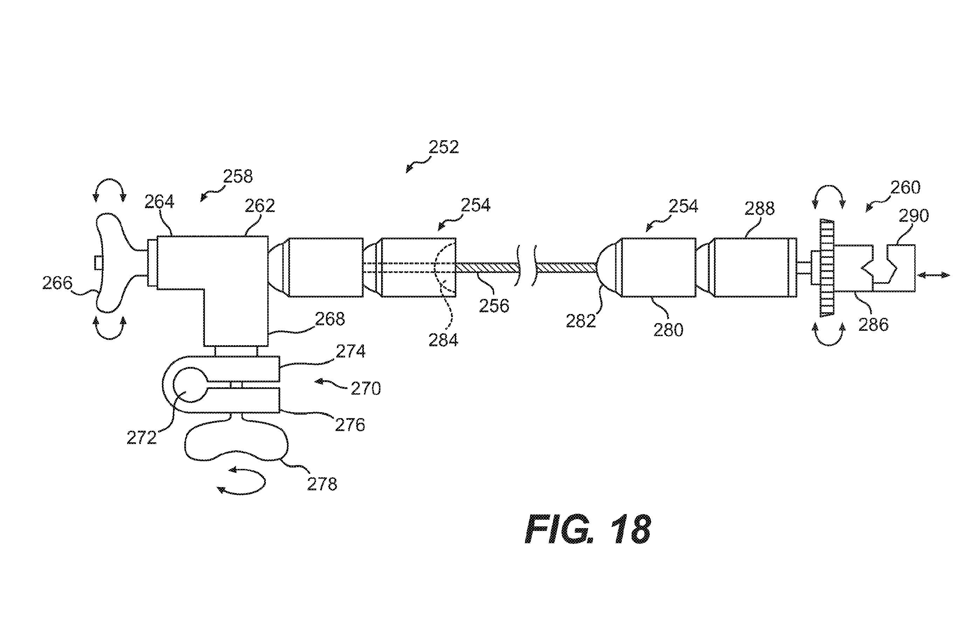

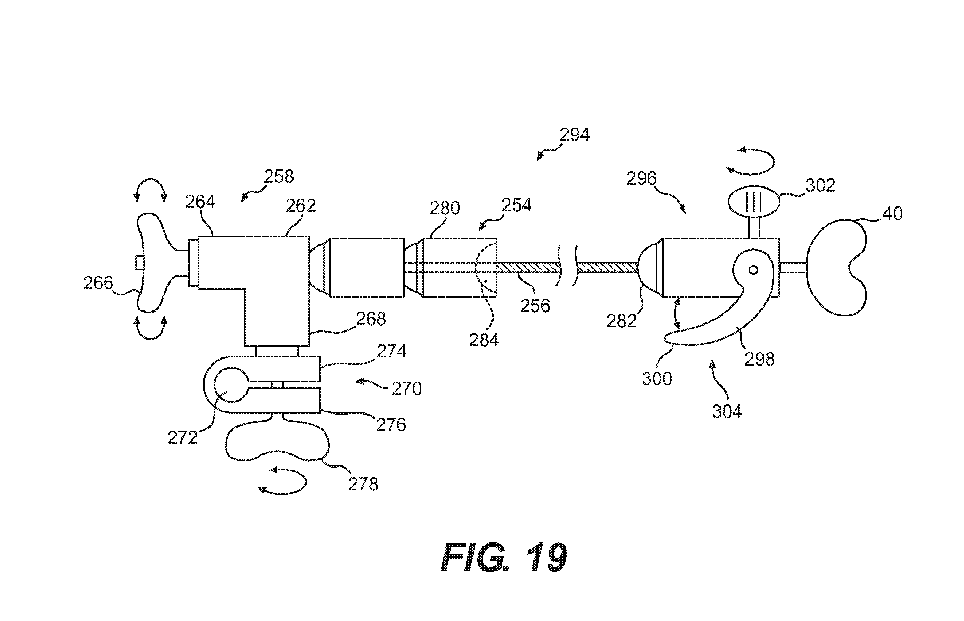

Reference is now made to Figs. 18 and 19, which illustrate two contemplated embodiments for the flexible shaft 42, 50, 58, 66 of the present invention. The description of these contemplated embodiments of the flexible shaft 42, 50, 58, 66 is not intended to be limiting of the invention.

As illustrated in Fig. 18, a flexible shaft 252 includes a plurality of individual segments 254 that are threaded onto a cable 256. The cable 256 extends between a first clamp 258 and a second clamp 260.

In the embodiment illustrated in Fig. 18, the first clamp 258 includes an L-shaped body 262. A first leg 264 extends along the axis of the cable 256. The first leg 264 includes a tightening nut 266. The tightening nut is threadedly disposed within the L-shaped body 262 so that turning of the nut 266 in one direction draws the cable 256 into the L-shaped body 262. Turning the nut 266 in the opposite direction releases the cable 256 from the L-shaped body 262. As should be apparent, when the cable 256 is drawn into the L-shaped body 262, the cable 256 compresses the segments 254 together. As a result, via friction, the segments 254 become fixed in a predetermined orientation, permitting the flexible shaft 252 to retain the predetermined orientation. When the nut 266 is loosened, the cable 256 is extended with respect to the segments 254, permitting the segments to move relative to one another.

The second leg 268 of the L-shaped body 262 has a clamp body 270 attached thereto. The clamp body 270 is essentially a U-shaped structure with a cylindrical opening 272 at one side and two opposed legs 274, 276 at the other. A nut 278 threadedly engages the opposed legs 274, 276. As should be apparent, when the nut 278 is tightened, the legs 274, 276 are brought into close proximity (or into contact) with one another. As a result, the diameter of the opening 272 is reduced so that the L-shaped body 262 maybe fixedly held on a support or other structure. When the nut 278 is loosened, the clamp 270 may be removed from a supporting structure.

Each of the segments 254 includes a cylindrically-shaped end 280 and a spherically-shaped end 282. The spherically-shaped end 282 of each of the segments 254 engages an indentation 284 within the end of the cylindrically-shaped end 280 of the adjacent segment 254, as shown. With this configuration for each of the segments 254, it is possible to manipulate the flexible arm 252 into virtually any configuration. Once the nut 266 is tightened, the segments 254 frictionally engage one another, thereby causing the flexible arm 252 to become rigid and retain the predetermined configuration.

In connection with this embodiment, it is contemplated that the spherically-shaped ends 282 and the indentations 284 may present roughened surfaces that improve frictional engagement therebetween. It is also contemplated that only one of the spherically-shaped ends 282 or the indentations 284 may be roughened to provide enhanced the frictional engagement between the components. Still other frictional enhancements may be employed including, but not limited to tacky materials, such as releasable adhesives and the like.

As should be apparent, the clamp 258 is intended to attach to a suitable structure, such as the support 22 discussed in connection with Fig. 1, for example.

The opposite end of the flexible arm 252 may include a clamp 260 that is configured to grasp a device, such as the cricoid manipulator head 40, the tool rack head 48, or the cheek manipulator head 56, etc.

In the illustrated embodiment, the clamp 260 includes a body 286 that is connected to the terminal (or end) segment 288 of the flexible arm 252. The body includes a movable segment 290 and a rotatable knob 292. Rotation of the knob 292 causes the moveable segment 290 to move relative to the body 286. This permits items to be held by the clamp 260.

Fig. 19 provides an alternative embodiment to the embodiment illustrated in Fig. 18.

The embodiment illustrated in Fig. 19 is presented, primarily because a practitioner typically would prefer to have a one-handed operation for the flexible shaft 252. One reason for this is that the practitioner's other hand typically is occupied with activities concerning the patient. For example, the practitioner may be holding a laryngoscope with the left hand and wish to apply external neck pressure via the cricoid manipulator head 40 with the right hand. If so, one disadvantage with the embodiment discussed in connection with Fig. 18 is the need for tightening of the nut 266, which is positioned a distance away from the patient. The practitioner would have to release the manipulator head 40 to tighten the nut 266, thus allowing the head 40 potentially to change position before being locked into place.

The flexible shaft 294 presents one contemplated embodiment for single-handed operation thereof.

The flexible shaft 294 that is illustrated in Fig. 19 includes many of the same components that are discussed in connection with Fig. 18. The differences, which are apparent in the illustration, concern the terminal segment 296.

The terminal segment 296 on the flexible shaft 294 includes a lever 298 that may be ratcheted into position to tighten the cable 256 and, thereby, secure the flexible shaft 294 in a predetermined orientation. The lever 298 is contemplated to be biased, by a spring, into a position where the end 300 is displaced a predetermined, axial distance from the terminal segment 296. Application of pressure to the lever 298 causes the cable 256 to be drawn into the terminal segment 296. It is contemplated that a single squeeze of the lever 298 will be sufficient to cause the flexible shaft 294 to become rigid so that it retains its desired orientation.

It is also contemplated that the lever 298 will be provided with a suitable release mechanism so that the flexible shaft 294 may be manipulated into any of a number of predetermined positions.

As illustrated, the terminal segment 296 includes a key 302, which may be rotated to secure a device, such as the cricoid manipulator head 40 in a suitable orientation.

In this embodiment, it is contemplated that the practitioner will hand tighten the nut 266 until the flexible shaft 294 is in a nearly rigid condition, suitable for use. Once the cricoid manipulator head 40 is positioned to apply suitable pressure to the cricoid cartilage, the lever 298 will be depressed to that the flexible shaft assumes a static position in a rigid condition.

It is contemplated for this embodiment, that the nut 266 will provide a coarse adjustment of the rigidity of the flexible shaft 294 and that the lever 298 will provide a fine adjustment of the rigidity of the flexible shaft 294.

As should be apparent to those skilled in the art, there are numerous ways in which the lever 298 may be designed to operate to provide the final adjustment of the rigidity for the flexible shaft 294. As such, further details concerning the construction of the lever 298 are not provided.

As should be appreciated by those skilled in the art, the lever 298 and its associated components form a distal locking mechanism 304. The distal locking mechanism 304 permits a releasable locking of the flexible shaft 294 to maintain the flexible shaft 294 in a selected, predetermined orientation. Specifically, the distal locking mechanism 304 permits the practitioner to place the flexible shaft 294 into a rigid condition with a one-handed operation, thereby facilitating manipulation of the flexible shaft 294.

For purposes of the discussion of the present invention, the term "rigid" has been used. It is contemplated, however, that the flexible shaft 294 need not be placed into a perfectly rigid condition. To the contrary, at least a semi-rigid condition will suffice in many instances to accomplish the objective of applying a suitable force to the cricoid cartilage without the practitioner having to release cricoid pressure. Whether locked into a rigid or semi-rigid condition, it is contemplated that the flexible shaft 294 will present sufficient resistance to forces to maintain adequate pressure on the cricoid cartilage, if used for this purpose.

While the locking mechanism 304 is discussed in connection with the embodiment illustrated in Fig. 19, the distal locking mechanism 304 may be employed with any of the embodiments described herein, as should be apparent to those skilled in the art.

It is noted that the components of the flexible shaft 252 and the flexible shaft 294 are contemplated to be made from steel, such as stainless steel. However, other materials may be employed without departing from the scope of the present invention.

As discussed above, the present invention is not intended to be limited to the specific embodiments described herein. To the contrary, there are numerous equivalents and variations that may be appreciated by those skilled in the art upon understanding the details presented herein. The present invention is intended to encompass the equivalents and variations that would be appreciated by those skilled in the art.

## Claims

1. A medical assistance apparatus, comprising:
a support adjacent to a patient;
a flexible shaft with a first end and a second end, with the first end being connected to the support;
a housing connected to the second end of the flexible shaft;
a shaft movably disposed with respect to the housing;
an adjustment device associated with the housing, permitting adjustment of the shaft with respect to the housing; and
a cricoid manipulating head connected to the shaft,
wherein the cricoid manipulating head is adjusted with respect to the housing along when the shaft is adjusted with respect to the housing.

2. The medical assistance apparatus of claim 1, wherein the support is a rail connected to a patient table.

3. The medical assistance apparatus of claim 1, further comprising:
a locking mechanism disposed on the flexible shaft, permitting the flexible shaft to be releasably locked into at least one of a semi-rigid or rigid state.

4. The medical assistance apparatus of claim 1, wherein the flexible shaft comprises at least one of a flexible material, a plurality of articulated segments, or a combination of a flexible material and at least one articulated segment.

5. The medical assistance apparatus of claim 1, further comprising:
a ball joint connecting the flexible shaft to the housing, thereby permitting multi-axial movement of the housing with respect to the flexible shaft.

6. The medical assistance apparatus of claim 1, wherein the adjustment device further comprises:
a plurality of tooth-engaging grooves disposed on the shaft;
a gear rotationally disposed adjacent to and engaging the plurality of tooth-engaging grooves;
a gear shaft on which the gear is disposed, the gear shaft being disposed rotationally with respect to the housing; and
a head disposed on the gear shaft, permitting manipulation of the shaft and the gear, thereby causing movement of the shaft and the cricoid manipulating head with respect to the housing.

7. The medical assistance device of claim 1, wherein the adjustment device further comprises:
threads on the shaft;
a threaded opening in the housing into which the shaft is threadedly disposed; and
a joint disposed at one end of the shaft, connecting the shaft to the cricoid manipulating head,
wherein the joint permits the cricoid manipulating head to rotate with respect to the shaft, independently of any rotation of the shaft within the housing.

8. The medical assistance device of claim 1, wherein the adjustment device further comprises:
a plurality of grooves disposed on the shaft;
a first adjustment lever and a second adjustment lever pivotally connected to the housing and disposed adjacent to substantially opposite sides of the shaft;
rounded ends on each of the first and second adjustment levers; and
a plurality of teeth disposed on the rounded ends of the first and second adjustment levers, the teeth engaging with the grooves on the shaft,
wherein actuation of the first and second adjustment levers causes the shaft to move in relation to the housing.

9. The medical assistance device of claim 1, wherein the cricoid manipulating head comprises a body with a hemi-cylindrical shape.

10. The medical assistance device of claim 1, wherein the cricoid manipulating head comprises:
a body defined by a central region, a right side lobe, and a left side lobe, wherein the body compliments a natural shape of a person's neck.

11. The medical assistance device of claim 1, wherein the cricoid manipulator head comprises:
a body defined by a central section with four flexible fingers extending therefrom.

12. A medical suite, comprising a medical assistance apparatus according to claim 1 and at least manipulator selected from a manipulator group comprising:
a cheek manipulator comprising a flexible shaft with a first end and a second end, with the first end being connected to the support, an L-shaped body movably connected to second end of the flexible shaft, the L-shaped body having a first leg and a second leg, wherein the first leg is movably disposed with respect to the flexible shaft and the second leg is provided to engage a patient's cheek, and an adjustment device associated with the flexible shaft, permitting adjustment of the first leg of the L-shaped body with respect to the flexible shaft,
a tongue manipulator comprising a flexible shaft with a first end and a second end, with the first end being connected to the support, a clamp attached to the second end of the flexible shaft, the clamp having first and second clamping members that oppose one another, and a tongue depressor disposable between the first and second clamping members of the clamp,
a tool rack comprising a flexible shaft with a first end and a second end, with the first end being connected to the support, and a tool rack head disposed on the second end of the flexible shaft, wherein the tool rack head comprises a body with a plurality of flexible fingers attached thereto, the fingers being disposed adjacent to one another so that items may be inserted therebetween, the fingers comprising at least a flexible surface thereon so that items inserted therebetween are retained therebetween at least partially due to the flexible surface on the plurality of fingers, and
a neck manipulator comprising a flexible shaft with a first end and a second end, with the first end being connected to the support, and a neck manipulator head fixedly disposed on the second end of the flexible shaft.

13. The medical suite of claim 12, wherein at least two manipulators are selected from the manipulator group.

14. The medical suite of claim 12, wherein at least three of the manipulators are selected from the manipulator group.

15. The medical suite of claim 12, wherein the support is a rod, substantially vertically oriented adjacent to a table.

16. The medical suite of claim 15, wherein the rod movably connects to a rail connected to a patient table.

17. A medical assistance device, comprising:
a support adjacent to a patient;
a flexible shaft with a first end and a second end, with the first end being connected to the support;
an L-shaped body movably connected to second end of the flexible shaft, the L-shaped body having a first leg and a second leg, wherein the first leg is movably disposed with respect to the flexible shaft and the second leg is provided to engage a patient's cheek; and
an adjustment device associated with the flexible shaft, permitting adjustment of the first leg of the L-shaped body with respect to the flexible shaft.

18. A medical assistance device, comprising:
a support adjacent to a patient;
a flexible shaft with a first end and a second end, with the first end being connected to the support;
a clamp attached to the second end of the flexible shaft, the clamp having first and second clamping members that oppose one another; and
a tongue depressor disposable between the first and second clamping members of the clamp.

19. A medical assistance device, comprising:
a support adjacent to a patient;
a flexible shaft with a first end and a second end, with the first end being connected to the support; and
a tool rack head disposed on the second end of the flexible shaft, wherein the tool rack head comprises a body with a plurality of flexible fingers attached thereto, the fingers being disposed adjacent to one another so that items may be inserted therebetween, the fingers comprising at least a flexible surface thereon so that items inserted therebetween are retained therebetween at least partially due to the flexible surface on the plurality of fingers.

20. A medical assistance device, comprising:
a support adjacent to a patient;
a flexible shaft with a first end and a second end, with the first end being connected to the support; and
a neck manipulator head fixedly disposed on the second end of the flexible shaft, wherein pressure may be applied by the neck manipulator head to a posterior surface of a patient's neck.
